# EUROPEAN PATENT SPECIFICATION

(11) **EP 1 363 617 B1**
(45) Date of publication and mention of the grant of the patent: **03.09.2008**
(21) Application number: 02740028.2
(22) Date of filing: 16.01.2002
(51) Int. Cl.: A61K 31/275, A61K 31/78, A61K 31/785, A61P 17/00, A61P 31/22, A61K 31/10

(54) **CYANOACRYLATE COMPOSITIONS FOR THERAPEUTIC TREATMENT OF WOUNDS REQUIRING REVISION**
CYANOACRYLAT-ZUSAMMENSETZUNGEN FÜR DIE THERAPEUTISCHE BEHANDLUNGVON WUNDEN, DIE EINE REVISION ERFORDERN
COMPOSITIONS DE CYANOACRYLATES POUR LE TRAITEMENT THERAPEUTIQUE DE PLAIES QUI NECESSITENT UNE REVISION

(30) Priority: 16.01.2001 DK 200100067; 16.01.2001 DK 200100068; 16.02.2001 DK 200100264
(43) Date of publication of application: 26.11.2003
(73) Proprietor: Lyster, Hans Brinch, 2770 Kastrup (DK)
(72) Inventor: Lyster, Hans Brinch, 2770 Kastrup (DK)
(74) Representative: Holme, Edvard
(86) International application number: PCT/DK2002/000034
(87) International publication number: WO 2002/062331

(56) References cited:
- EP-A1- 0 323 652
- WO-A1-01/12243
- WO-A1-93/20829
- WO-A1-93/25196
- WO-A1-95/00153
- WO-A1-97/47310
- WO-A1-98/03152
- US-A1- 2001 051 179
- J.A. FOGLE ET AL.: 'Tissue adhesive arrests stromal melting in the human cornea' AMERICAN JOURNAL OF OPHTHALMOLOGY vol. 89, no. 6, 1980, pages 795 - 802, XP002909981
- DATABASE CAPLUS [Online] DOC. NO. 122:114859 HOWELL, JOHAN M. ET AL.: 'Comparison of effects of suture and cyanoacrylate tissue adhesive on bacterial counts in contaminated lacerations', XP002909982 Retrieved from STN Database accession no. 1995:354182 & ANTIMICROB. AGENTS CHEMOTHER. vol. 39, no. 2, 1995, pages 559 - 560
- DATABASE CAPLUS [Online] DOC. NR. 101:83795 GALIL, K.A. ET AL.: 'The healing of hamster skin ulcers treated with n-butyl-2-cyanoacrylate (histoacryl blue)', XP002909983 Retrieved from STN Database accession no. 1984:483795 & J. BIOMED. MATER. RES. vol. 18, no. 6, 1984, pages 601 - 607
- DATABASE CAPLUS [Online] DOC. NR. 135:97379 BAZELL, GREGORY M. ET AL.: 'Reduction mammoplasty incision closure with octyl-2-cyanoacrylate', XP002909984 Retrieved from STN Database accession no. 2001:119967 & SURGICAL FORUM vol. 51, 2000, pages 611 - 613

## Description

The invention relates to the use of at least one cyanoacrylate having the general monomer formula I: 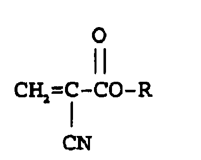 for producing a medicament for therapeutic treatment of wounds requiring revision.

WO 95/00153 describes cyanoacrylate adhesives which are incorporated in the present patent application by preference. The acrylate adhesives are used for spraying or covering a larger or smaller skin area which is especially exposed to friction and/or irritation. Friction and/or irritation of especially exposed skin areas is inhibited by covering the areas with a protective cyanoacrylate polymer coating. Thereby, friction contact with the selected skin areas is prevented and the risk of deep and slowly healing lesions being formed is reduced.

WO 97/47310 and WO 98/03152 describe a glyceryl poly(meth)acrylate gel for treating or preventing different skin disorders. In case of candida infections, Herpes Simplex 1 and 2 infections, sunburns or irritation of oral mucous membrane, the water absorbing and osmotic properties of this gel will contribute to the dehydration of micro organisms present which therefore no longer will be able to survive.

US 5,306,490 discloses the coating of a skin area with a cyanoacrylate polymer coating for preventing blister formation.

WO 93/25196 discloses the gluing together of edges of skin which cannot or only can be sutured with difficulty, and this application largely takes place in the hospital emergencies. Another adhesive cyanoacrylate-based composition is known form WO 01/012243.

Treatment of corneal lesions is described in "Tissue adhesive arrest stromal melting in the human cornea", J.A. Fogle, M.D., Kenneth R. Kennyon, M.D., and C. Stephen Foster, M.D., Am. J. Ophthalmol. 1980, vol. 89, no. 6, pp. 795-802, in which an isobuthyl-2-cyanoacrylate was applied together with bandage lens following surgical removal of sick tissue and supplemented with subsequent medical post-surgical treatment. In this case, the cyanoacrylate polymer coating serves as plaster under which the wound is allowed to heal during a long period.

US 4,752,472 discloses cosmetic removal of smaller impurities and dead cells from the skin surface and especially from sebaceous follicles in the skin. Cyanoacrylate adhesive is spread over the area desired cleaned. The impurities are attached to the polymer coating which can be pulled off after a short time in order to thereby cosmetically remove impurities from the skin surface.

A wound is defined by the expert as an open lesion of exterior and interior surface of an organism, for example a lesion of the skin on a human. More than 30,000 Danes suffer constantly of acute or chronic wounds, and treatment of wounds is estimated to cost the Danish health service between 1 and 2 billion kroner annually.

Types of wounds in humans can be classified as wounds with tissue loss on the surface of the body, and divided primarily into acute wounds and chronic wounds. The acute wounds heal up normally and often without complications, where the chronic wounds which are caused by an underlying disease have a very slow healing which often stop completely. Especially large, acute and chronic wounds are very difficult to keep clean, and such wounds can easily be infected with microorganisms, such as bacteria, fungi and vira, which multiply and invade either the deeper part of the wound, the wound edges and possibly the wound surroundings, which inhibit the healing and necessitate revision of the wound. Also smaller chronic wounds in patient with reduced or poor immune defence heal slowly and therefore often get infected.

The acute wounds, vulvus, are produced after exterior trauma and comprise surgical wounds and infections-conditioned wounds, for example after operation, traumatic wounds, such as stab wounds, abrasions, burns, etching, and frost-bites.

The chronic wounds, *ulcus*, are caused by a disease process and comprise for example pressure sores, bedsores, venous- or arterial-conditioned wounds or combination wounds, of these wounds, wounds caused by diabetes mellitus, infected wounds or fistulas.

A wound is further divided into the types black, yellow and red wounds, and it is this division that is applied in relation to determination of local treatment.

The black wound is the most serious and is recognised by its black necroses which are found in and/or is covering the wound. These result in a delayed healing through inhibition of epithelization, ingrowth of fibroblasts to connective tissue healing and an increased collagenosis. Finally, an increased bacterial growth can occur during a necrosis resulting in infection. Black necroses at arterial and diabetic wounds are especially seen in case of wounds related to vessel changes. The treatment is most often removal of the necrosis by surgical wound revision. As it is a painful process to revise a wound, the physician will in many cases defer the revision until the necrosis will get loosen at the edges by itself or await a spontaneous exfoliation. Alternatively, the wound revision must often take place using a form of anaesthesia.

The yellow wound consists of changed fatty tissue, connective tissue residues and fibrine precipitation. This type of wound, which is most frequently seen in arteriosclerotic and diabetic wounds but rarely in venous wounds, are generally less important to the healing than the black wound. The treatment can comprise wound cleaning, possibly with the addition of a wound revision when infections supervene.

The red wound primarily consists of granulation tissue, the appearance of which has named the wound. After correct treatment, black and yellow wounds will reach this phase in which the wound usually heals quickly, and the wound revision aspires to promote formation of the read wound.

Revision of a wound comprises removal, most often surgical, of the crust and unhealthy surrounding tissue, such as necrotic tissue. The wound revision is often supplemented with a prophylactic and/or therapeutic treatment, such as with antibiotics or antifungal drug.

An aspect of the invention is to provide a medicament of the kind mentioned in the opening paragraph, by means of which revision of a patient's wounds can be done more quickly, easily and less expensively and without painful nuisances to the patient.

The novel and unique features according to the invention, whereby this is achieved, is the fact that for producing the medicament is used a cyanoacrylate having the general monomer formula I, in which R preferably is chosen from the group of
alkyls or alkenyles having 1 to 10 carbon atoms,
cycloalkyls having 5 to 10 carbon atoms,
phenyl,
2-ethoxyethyl,
3-methoxybutyl,
arenes or alkyl-substituted arenes
or a substituent
having the formula II: 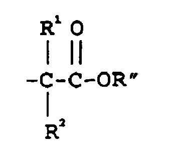 in which
R¹ and R² are chosen independently of each other from the group consisting of hydrogen, methyl, ethyl, propyl or butyl, and
R'' is chosen from the group consisting of alkyls or alkenyles having 1 to 10 carbon atoms, cycloalkyls having 3 to 10 carbon atoms,
or R" is chosen from the group consisting of phenyl, benzyl, methylbenzyl, phenylethyl or halogen- substituted or alkyl-substituted compounds of these.

Said cyanoacrylate compounds and compositions containing different of said cyanoacrylate compounds have not previously formed part of medicaments for revision of acute or chronic wounds. The chronic wounds especially require treatment when the wounds are found as black, necrotic wounds. Often acute wounds develop into chronic wounds which necessitates wound revision. In case of e.g. burns, the additional advantage is obtained in that application of a cyanoacrylate polymer coat contributes to prevent the loss of often large amounts of fluid.

The liquid cyanoacrylate compound has the advantage that it, when spread over a crust, can penetrate into and be distributed into the cavities of the crust. Upon contact with the tissue fluid, the polymerization of the cyanoacrylate monomers begins which gradually creates a polymer network and a coat that adhere to the crust effectively, unbreakably and securely.

By means of a cyanoacrylate polymer coat which will be formed after complete polymerization which will be completely completed already after 1 minute for most cyanoacrylate compounds and combinations of cyanoacrylate compounds, even a relatively thick crust can be pulled off easily and quickly, and the exposed, open lesion can be cleaned out and aftertreated further according to individual needs and discretion. The reaction time depends on the applied amount of cyanoacrylate and the surface of the wound, and can preferably be as short as under 10-15 seconds.

The wound can easily be rinsed and cleaned out by means of conventional cleansing methods, and possible wound debris, contaminates or other impurities can be removed by means of a supplementary treatment with the cyanoacrylate medicament and subsequent pulling off of these components by means of the formed polymer coat.

The polymer can only adhere very loosely to healthy tissue, and the cyanoacrylate medicament can preferably be placed across the wound in such a way that the hardened polymer coat is extending beyond the limitations of the crust or wound in over the healthy skin so that a detachable edge region can serve as gripping region during the pulling off of the crust or components desired removed. A residue of the polymer coat can possibly be left as an especially advantageous protection of the wound edge.

As medicaments, as mentioned earlier, only partly adhere to healthy tissue, wounds contaminated with various foreign bodies such as e.g. glass splinters, remains of suture material or gunpowder burns, can be cleaned out easily and painlessly.

A péans damages the tissue around which it is clamped and in replacement of such a péans, a polymer coat formed by the medicament according to the invention can be used to grip two wound edges and retain them while they are drawn together so that they can be sutured.

The medicament can e.g. advantageously be used in connection with removal of wounds covering sutures which are to be removed from a surgical incision. Also undesirable hair growth in a region around a wound, or hair desired removed prior to the making of a surgical incision can easily be removed. The polymer coat will settle around the foreign bodies such ad the glass splinters or the undesirable hairs respectively and capture and retain these for subsequent removal when the polymer coat is pulled off the wound.

When using the medicament according to the invention for revision of wounds, the patient can advantageously avoid a surgical operation which is time-consuming to both patient and surgeon as removal of crusts by means of cyanoacrylate compounds and compositions of these can take place without use of surgery. In by far the most cases, the preceding local anesthesia which often is necessary in case of surgical wound revision can advantageously be spared when the present medicament is used for wound revision.

After pulling off of the crust, a possible antibiotic, fungicidal drug, or drug for treating eczema can be applied to the revised wound and subsequently a new cyanoacrylate polymer coat can possibly be applied to cover the treated wound in replacement of a conventional bandage or conventional plaster.

It is especially preferred to use a cyanoacrylate polymer coat for protecting a revised, possibly further treated wound on sites where it is difficult to fasten a bandage or a plaster, such as e.g. around articulations and under mammae, or in situations where it will be an advantage that the bandage can follow the movements of the body unobstructedly without being removed from the wound.

The cyanoacrylate compounds do not adhere to vital skin/tissue, and the difference between the adhesion ability to vital skin/tissue and the attack of skin/tissue forms a joint basis of the treatment of the above diseases.

A large number of the above-mentioned different substituents known per se R, R¹, R² and R'' can be used, and they can all form part of a cyanoacrylate polymer for producing a medicament according to the present invention.

The medicament can be produced by using a single cyanoacrylate compound or the medicament can comprise a combination of cyanoacrylates in which optionally R, R¹, R² and R'' can be identical or different.

Preferably, a simple, inexpensive ethyl cyanoacrylate compound is used for producing the medicaments according to the invention.

Such an inexpensive, liquid ethyl cyanoacrylate compound is for example commercially available from Loctite European Group, Arabellastrasse 17, D-81925 Munich, Germany, under the name LOCTITE 411. Ethyl cyanoacrylate of this kind is sold for adhering e.g. plastic parts for medical equipment. Another commercially available and applicable cyanoacrylate compound is sold under the commercial name LOCTITE SUPER ATTAK.

Cyanoacrylate compounds have previously been used for gluing wounds together, and there is therefore no health risk in using cyanoacrylate compounds for this new application.

Advantageously, the medicament can furthermore comprise one or more additives.

A stabilizer which can prevent the medicament from polymerizing spontaneously during storage can extend the life of the medicament. Stabilizers having a pH equal to or under 7 and which can be neutralised upon contact with moisture is especially preferred.

In some cases, it can also be an advantage to add an agent such as an alkane, a ketone or an alcohol having C1-C10 which can accelerate the polymerization reaction so that the formation of the polymer coat can take place within merely a few seconds.

LOCTITE 41 contains both a stabilizer and an agent for accelerating the polymerization reaction.

The medicament containing the cyanoacrylate compound can either be liquid or in form of a gel. The gelling properties can be provided by e.g. letting the monomers polymerise partly to e.g. di- or trimers in order to thereby make the medicament more viscous and thereby more suitable to settle closely and locally over the area requiring treatment which may have a variable topography and morphology without problems.

For many of the applications, the medicament can advantageously be added an inert colorant so that it is possible to easily locate the limits of the polymer coat when e.g. a crust is to be pulled off.

The present invention will be described in details with reference to the subsequent examples.

### EXAMPLES

All patients participated after informing guidance of their own free will in the tests mentioned below. The treatment with the medicament according to the invention was done by the inventor who is a qualified medical specialist in general medicine and has practised as medical specialist and district medical head doctor in Osby, Sweden.

Examples 1-3 are comparative examples of patients treated with ethyl cyanoacrylate (Loctite Super Attak^{®}, Henkel Loctite Adhesive Ltd.) and conventional treatment respectively.

### Example 1

### Comparative study of skin removal from feet

Four patients (S1-S4) had skin removed from wounds requiring treatment on one foot with cyanoacrylate treatment and on the other foot with conventional dermatological treatment in form of filing off and bandaging. When not obtaining success by conventional treatment, cyanoacrylate treatment continued. The results are given in Table I below.

**Table I**

| **Patient no.** | **Sex/age** | **Treatment location** | **Treatment form** | **Duration of treatment** | **Treatment result** |
|---|---|---|---|---|---|
| S1¹⁾ | man/68 | Right foot | cyanoacry-late application | about 3 times daily, addi-tional dally treatments as requited at e.g. personal hygiene or pain | distinct healing after 1 week's treatment, skin normalized, still symptom free without fissures after 6 months on sustained filing off of callous skin |
| S2²⁾ | woman/7 5 | Left foot 1^{st} treat-ment | skin removal by filing off skin cal-losity followed by bandaging | as required | no visible result after 1 week |
| S3²⁾ | man/83 | Left foot 2^{nd} Left foot 2^{nd} treat-ment (following 1^{st} treat-ment) | cyanoacry-late application | as right foot | as right foot |
| S4²⁾ | man/78 | Right foot | cyanoacry-late application | about 3 times within 24 hours | Removal of skin corresponding to an extent allo-wing painless cleansing of fissure. Subsequently quick healing |

| | | | | | |
|---|---|---|---|---|---|
| 1) Type 2 diabetes and severe callosity of skin on both feet especially on pads; in addition fissures and light haemorrhages. 2) Diabetes and severe callosity of skin on both feet especially on pads; in addition fissures and light haemorrhages. 3) Diabetes. Deeply infected fissure on lateral part of right foot. The foot had previously been treated by medical specialist with antibiotics per os and locally but without effect. The depth of the fissure did not allow for cleaning out the infection without great pain. | | | | | |

Patients S1 and S3 displayed all identical positive treatment response in relation to dermatological treatment, and the results are therefore jointly given in Table II above.

Patients S1 and S4 had severe pains when the pads were loaded during walking. The pains disappeared after healing. A simple method for skin removal and cleaning out of fissures is given here. The method is simple and painless and has a convincing effect as shown in Table II. It is to be noted that the sooner callous skin is removed, the smaller the risk of fissures being generated, of a possible infection arise in the fissures, and of this infection possibly spreading.

### Example 2

### Initial test: Removal of crust from small superficial wounds

Wounds of six patients with wounds of 1-2 cm x 1-2 cm were painted with cyanoacrylate. The crust was removed after polymerization easily and with no pain in all patients. No larger haemorrhages observed but a few and sparse between 0,5mm x 0,5mm, spot small haemorrhages. Two of the patients had additional wounds which were removed surgically for comparison. Pain and larger haemorrhages observed.

### Example 3

### Revision of superficial wounds on crus

Seven patients (R1-R7) with aetiologically different, not or only a little infected wounds on crus were treated with cyanoacrylate without prior anaesthesia. The results are given in Table II-below.

All patients had previously had similar wounds, for which they had been treated with surgical removal of crust by means of a scalpel and tweezers during preceding medication with Diazepam^{®} per os and Ketogan^{®}.

**Table II**

| **Patient No.** | **Aetiology** | **Wound size/ duration before cyanoacrylate treatment** | **Treatment** | **After-treatment/ Treatment result** |
|---|---|---|---|---|
| R1¹⁾ ♀, 78 years | Cardiovas-cular disease oedema | 5cmx6cm, slight Infection/ about 6 months | Previously tried: Fucidin compress, anti-biotics per os. Wound removal with scalpel not possible due to pain. Alternatively thereafter: 1 cyanoacrylate application, pulling off of crust. Cleansing | 1 add. cyanoacry-late application to remove necrotic tissue/ Frequent bandage change. Wound completely healed after three weeks |
| R2" ♂, 82 years | Cardiovas-cular disease | 5cm×8cm/ for about 6 months | Previously tried: Fucidin ointment and bandaging Alternatively thereafter 1 cyanoacrylate application, pulling off of crust. Cleansing | Uke **R1** |
| R3¹⁾ ♀, 84 years | Cardiov as-cular disease | about 5cm in diameter/about 8 months about 8 | Like R2 | Like R1 |
| R4¹⁾ ♀, ♀, 76 years | Type II dia-betes overweight | 3-5xcm x 3-5cm/ about 12 months | Like R2 | Frequent bandage changes. After one week, distinct granulation. Total healing after 6 weeks |
| R5¹⁾ ♀, 84 year | Type II dia-betes overweight | 3-5xcm x 3-5cm/ about 12 months | Uke R2 | Frequent bandage changes. After one week, distinct granulation. Total healing after 6 weeks |
| R6¹⁾ ♂, 72 years | Type I diabetes, overweight | 3-5xcm x 3-5cm/ about 12 months about 12 months | Like R2 | Frequent bandage changes. After one week, distinct granulation. Total healing after 8 weeks |
| R7¹⁾ ♂, 80 years | Type I diabetes, overweight | 3-5xcm 3-5cm/ about 12 months | Like R2 | Frequent bandage distinct granulation. Total distinct granulation. Total healing after 7 weeks |
| R8 ♀, 65 years | Large mammae | Left: Irritation and humidity | Previously: None Thereafter: None | Careful personal hygiene Careful personal hygiene just like left mammae. Slow healing |
| | | Right: Suppurating wounds | Previously: None Thereafter: cyanoacrylate application 3 times a day for 3 days | Careful personal hygiene, healing began quickly to complete healing after 1 month |

| | | | | |
|---|---|---|---|---|
| 1) After cyanoacrylate treatment and the subsequent removal of crust, no bandage cont. medicine, such as antibiotics, chlorhexidine or hydrogen peroxide, were used. Wounds only kept clean with sterile saline water and frequent bandage changes. | | | | |

## Claims

1. Use of at least one cyanoacrylate having the general monomer formula I: 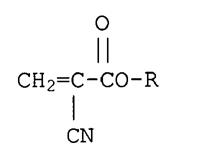 for producing a medicament for therapeutic treatment of wounds requiring revision,
in which R preferably is chosen from the group of alkyls or alkenyles having 1 to 10 carbon atoms,
cycloalkyls having 5 to 10 carbon atoms,
phenyl,
2-ethoxyethyl,
3-methoxybutyl,
arenes or alkyl-substituted arenes,
or a substituent
having the formula II: 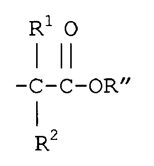 in which
R¹ and R² are chosen independently of each other from the group consisting of hydrogen, ethyl, propyl or butyl, and
R" is chosen from the group consisting of alkyls or alkenyles having 1 to 10 carbon atoms, cycloalkyls having 3 to 10 carbon atoms,
or R" is chosen from the group consisting of phenyl, benzyl, methylbenzyl, phenylbenzyl or halogen-substituted or alkyl-substituted compounds of these.

2. Use according to claim 1, wherein the medicament comprises a combination of cyanoacrylates, in which R is different.

3. Use according to claim 1, wherein R is ethyl.

4. Use according to claim 1, 2 or 3, wherein the medicament is used for revision of acute or chronic wounds which are covered by especially black or yellow crusts.

5. Use according to claim 1, 2 or 3, wherein the medicament is used for removal of undesired elements from a wound or a skin area.

6. Use according to claim 1, 2 or 3, wherein the medicament is applied in the area at the wound edges of a wound requiring suturing for forming gripping flaps for maintaining contact between wound edges during suturing.

7. Use according to any of the claims 1-6, wherein the at least one cyanoacrylate monomer polymerises upon contact with human, moist, dermal tissue or humane, moist mucosa.

8. Use according to any of the claims 1-7, wherein the medicament further comprises one or more additives chosen from the group consisting of stabilizers for preventing the medicament from polymerizing spontaneously during storage, agents for accelerating the polymerization reaction or a neutral colorant.

9. Use according to claim 8, wherein the stabilizer has a pH equal to or under 7 and is neutralized upon contact with moisture.

10. Use according to claim 8, wherein the agent for accelerating the polymerization reaction is an alkane, a ketone or an alcohol with C1-C10.

11. Use according to any of the claims 1-10, wherein the medicament is liquid.

12. Use according to any of the claims 1-10, wherein the medicament is a gel.

## Patentansprüche

1. Die Verwendung von mindestens einem Cyanoacrylat mit der generellen monomeren Formel I: 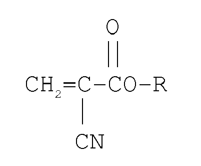 um ein Medikament für die therapeutische Behandlung von Wunden, die einer Revision bedürfen, herzustellen,
wobei R vorzugsweise ausgewählt ist aus der Gruppe von Alkylen oder Alkenylen, die 1 bis 10 Kohlenstoffatome haben, Cycloalkylen, die 5-10 Kohlenstoffatome haben, Phenyl, 2-ethoxyethyl, 3-methoxybutyl, Arene oder alkyl-substituierte Arene oder einem Substituenten der Formel II: 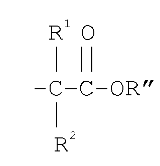 wobei
R1 und R2 unabhängig voneinander ausgewählt sind aus der Gruppe, die aus Wasserstoff, Ethyl, Propyl oder Butyl besteht, und
R" ausgewählt ist aus der Gruppe, die aus Alkylen oder Alkenylen mit 1 bis 10 Kohlenstoffatomen, Cycloalkylen mit 3 bis 10 Kohlenstoffatomen besteht, oder wobei R" ausgewählt ist aus der Gruppe, die aus Phenyl, Benzyl, Methylbenzyl, Phenylbenzyl, oder halogen-substituierten oder alkyl-substituierten Verbindungen dieser Klassen besteht.

2. Die Verwendung nach Anspruch 1, wobei das Medikament eine Kombination von Cyanoacrylaten umfasst, in welcher R unterschiedlich ist.

3. Die Verwendung nach Anspruch 1, wobei R Ethyl ist.

4. Die Verwendung nach einem der Ansprüche 1, 2 oder 3, wobei das Medikament für die Revision von akuten oder chronischen Wunden, die von besonders schwarzen oder gelben Krusten bedeckt sind, benutzt wird.

5. Die Verwendung nach einem der Ansprüche 1, 2 oder 3, wobei das Medikament für das Entfernen von ungewünschten Elementen aus der Wunde oder von der Hautfläche benutzt wird.

6. Die Verwendung nach einem der Ansprüche 1, 2 oder 3, wobei das Medikament auf die Fläche an den Wundrändern einer Wunde, die einer Naht bedarf, aufgebracht wird, um Greifklappen zu formen, um während des Nähens einen Kontakt zwischen Wundrändern aufrecht zu erhalten.

7. Die Verwendung nach einem der Ansprüche 1-6, wobei das mindestens eine Cyanoacrylatmonomer bei Kontakt mit menschlichem, feuchten Hautgewebe oder mit menschlicher, feuchter Schleimhaut polymerisiert.

8. Die Verwendung nach einem der Ansprüche 1-7, wobei das Medikament weiterhin eine oder mehrere Beimengungen umfasst, die aus der Gruppe ausgewählt ist, die aus Stabilisatoren, die eine spontane Polymerisation des Medikamentes während der Lagerung verhindern, Stoffen, die die Polymerisationsreaktion beschleunigen, oder einem neutralen Farbmittel besteht.

9. Die Verwendung nach Anspruch 8, wobei der Stabilisator einen pH Wert von 7 oder weniger hat, und bei Kontakt mit Feuchtigkeit neutralisiert wird.

10. Die Verwendung nach Anspruch 8, wobei der Stoff, der die Polymerisationsreaktion beschleunigt, ein Alkan, ein Keton oder ein C1-C10-Alkohol ist.

11. Die Verwendung nach einem der Ansprüche 1-10, wobei das Medikament flüssig ist.

12. Die Verwendung nach einem der Ansprüche 1-10, wobei das Medikament ein Gel ist.

## Revendications

1. Utilisation d'au moins un cyanoacrylate ayant la formule de monomère générale I : 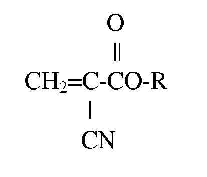 pour préparer un médicament pour le traitement thérapeutique de plaies nécessitant une révision,
dans laquelle R est de préférence choisis parmi le groupe des alkyles ou alcényles ayant 1 à 10 atomes de carbone, cycloalkyles ayant 5 à 10 atomes de carbone,
phényle,
2-éthoxyéthyle
3-méthoxybutyle,
arènes ou arènes alkyl-substitués,
ou un substituant ayant la formule II : 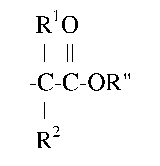 dans laquelle R¹ et R² sont choisis indépendamment l'un de l'autre parmi le groupe consistant en hydrogène, éthyle, propyle ou butyle, et
R" est choisi parmi le groupe consistant en alkyles ou alcényles ayant 1 à 10 atomes de carbone, cycloalkyles ayant 3 à 10 atomes de carbone,
ou R" est choisi parmi le groupe consistant en phényle, benzyle, méthylbenzyle, phénylbenzyle ou leurs composés halogéno-substitués ou alkyle-substitués.

2. Utilisation selon la revendication 1, dans laquelle le médicament comprend une combinaison de cyanoacrylates, dans laquelle R est différent.

3. Utilisation selon la revendication 1, dans laquelle R est un éthyle.

4. Utilisation selon la revendication 1, 2 ou 3, dans laquelle le médicament est utilisé pour la révision de plaies aiguës ou chroniques qui sont couvertes particulièrement par des croûtes noires ou jaunes.

5. Utilisation selon la revendication 1, 2 ou 3, dans laquelle le médicament est utilisé pour retirer des éléments non désirés d'une plaie ou d'une région de peau.

6. Utilisation selon la revendication 1, 2 ou 3, dans laquelle le médicament est appliqué dans la région des bords de la plaie d'une plaie nécessitant une suture pour former des pattes de préhension pour maintenir le contact entre les bords de la plaie pendant la suture.

7. Utilisation selon l'une quelconque des revendications 1 à 6, dans laquelle l'au moins un monomère cyanoacrylate polymérise lors du contact avec un tissu humain, humide, dermique ou une muqueuse humaine, humide.

8. Utilisation selon l'une quelconque des revendications 1 à 7, dans laquelle le médicament comprend en outre un ou plusieurs additifs choisis parmi le groupe consistant en stabilisants pour empêcher le médicament de polymériser spontanément durant le stockage, des agents pour accélérer la réaction de polymérisation ou un colorant neutre.

9. Utilisation selon la revendication 8, dans laquelle le stabilisant a un pH égal ou inférieur à 7 et est neutralisé lors du contact avec l'humidité.

10. Utilisation selon la revendication 8, dans laquelle l'agent pour accélérer la réaction de polymérisation est un alcane, une cétone ou un alcool en C1-C10.

11. Utilisation selon l'une quelconque des revendications 1 à 10, dans laquelle le médicament est liquide.

12. Utilisation selon l'une quelconque des revendications 1 à 10, dans laquelle le médicament est un gel.
